# EUROPEAN PATENT APPLICATION

(11) **EP 4 807 752 A1**
(43) Date of publication of application: **16.09.2026**
(21) Application number: 23958286.9
(22) Date of filing: 08.11.2023
(51) Int. Cl.: G16C 10/00

(54) **ENERGY CALCULATION PROGRAM, ENERGY CALCULATION METHOD, AND INFORMATION PROCESSING DEVICE**

(71) Applicant: Fujitsu Limited, Kawasaki-shi, Kanagawa 211-8588 (JP)
(72) Inventor: MATSUMOTO, Kazuhiro, Kawasaki-shi, Kanagawa 211-8588 (JP)
(74) Representative: Haseltine Lake Kempner LLP
(86) International application number: PCT/JP2023/040310
(87) International publication number: WO 2025/099885

(57) **Abstract**

An information processing device (100), using quantum chemical calculation, calculates states of a substance, based on values of parameters for expressing the states of the substance while changing the values of the parameters and calculates energies of the states based on the calculated states. The information processing device (100) assigns ranks to sets of the calculated energies of the states such that a set corresponding to a point relatively closer to an origin in a coordinate system including axes representing the energies of the states has a relatively higher rank. The information processing device (100) changes the value of the parameter based on the assigned rank and the value of the parameter corresponding to the rank, so that the rank of the set of energies becomes higher.

## Description

### TECHNICAL FIELD

The present invention relates to an energy calculating program, an energy calculating method, and an information processing device.

### BACKGROUND ART

In fields such as materials development and pharmaceutical development, computational chemistry is sometimes used to analyze the properties of candidate substances for materials or pharmaceuticals. When analyzing properties of a substance, it is necessary to analyze not only a ground state thereof but also excited states thereof to determine whether the substance reacts or how readily a reaction proceeds. The ground state is the lowest energy state that a substance can occupy energetically. The excited states are energy states with a higher energy than the ground state among the possible energy states of a substance. One method for analyzing the excited states of a substance is, for example, SSVQE (Subspace Search Variational Quantum Eigensolver).

Prior art includes, for example, methods for finding excited states of a Hamiltonian in a hybrid system including a quantum computer and a classical computer. There is also technology for calculating the structure of the ground state, the lowest excited singlet state, and the lowest excited triplet state of π-conjugated compounds contained in the light-emitting layer of an organic EL device. Furthermore, there is a technology for estimating the energies of the ground state and excited states of a fermionic Hamiltonian using a classically boosted variational quantum eigen solver (VQE).
Patent Document 1 International Publication No. 2020/090559
Patent Document 2 International Publication No. 2016/017684
Patent Document 3 US Patent Application Publication No. 2022/0284337 Specification

### DISCLOSURE OF INVENTION

### PROBLEM TO BE SOLVED BY THE INVENTION

However, in the prior art, it is difficult to calculate the energy of excited states when analyzing the properties of substances. For example, in SSVQE, it is necessary to set weight coefficients for the energy of each state, but setting appropriate weight coefficients is difficult.

In one aspect, the present invention aims to calculate the energy of excited states of a material.

### MEANS FOR SOLVING PROBLEM

In one embodiment, an energy calculating program for causing a computer to execute a process is provided, the process including: while changing a value of a parameter for expressing each of a plurality of states including a ground state and one or more excited states of a substance, calculating by quantum chemical calculation, the plurality of states, based on the value of the parameter; and calculating based on the calculated plurality of states, a plurality of energy sets of the plurality of states. The changing the value of the parameter includes: assigning a rank to each of the calculated plurality of energy sets so that in a coordinate system including axes representing the plurality of energy sets, the rank of a set that is among the plurality of energy sets and corresponds to a point relatively closer to an origin of the coordinate system is relatively higher, and changing the value of the parameter based on the assigned rank and the value of the parameter corresponding to the rank so that the rank of the set becomes higher.

### EFFECT OF THE INVENTION

According to one aspect of the present invention, an effect is achieved in that the energy of an excited state of a substance can be calculated.

### BRIEF DESCRIPTION OF DRAWINGS

Fig. 1 is an explanatory diagram depicting an example of an energy calculating method according to an embodiment.
Fig. 2 is an explanatory diagram depicting an example of an information processing system 200.
Fig. 3 is a block diagram depicting an example of a hardware configuration of an excitation energy calculating device 201.
Fig. 4 is an explanatory diagram depicting an example of energy calculation setting information.
Fig. 5 is a block diagram depicting an example of the functional configuration of the excitation energy calculating device 201.
Fig. 6 is an explanatory diagram depicting an example of stored contents of s calculation result table 600.
Fig. 7 is an explanatory diagram depicting an example of updating the stored contents of the calculation result table 600.
Fig. 8 is an explanatory diagram depicting an example of setting priorities.
Fig. 9 is an explanatory diagram depicting a specific example of energy analysis results.
Fig. 10 is a flowchart depicting an example of a procedure of an energy calculating process of the excitation energy calculating device 201.

### BEST MODE(S) FOR CARRYING OUT THE INVENTION

Herein, an energy calculating program, an energy calculating method, and an information processing device according to the present invention are described in detail with reference to the accompanying drawings,.

### (Embodiment)

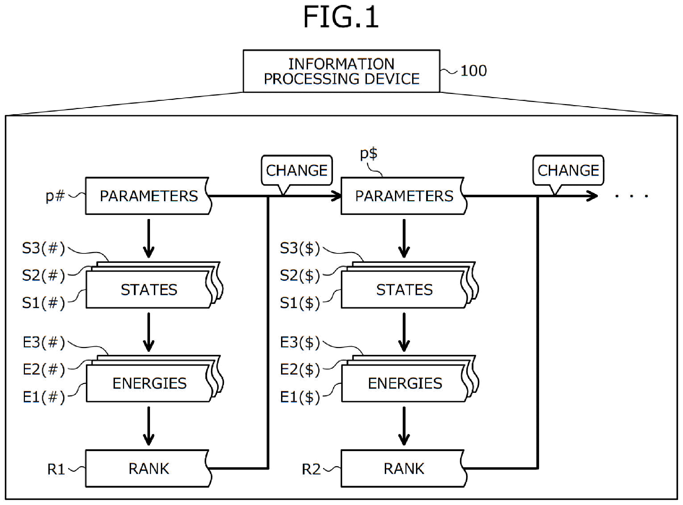

Fig. 1 is an explanatory diagram depicting an example of an energy calculating method according to an embodiment. In Fig. 1, the information processing device 100 is a computer that calculates the energy of an excited state of a substance (material). The information processing device 100 is, for example, a server or a personal computer (PC). The substance is, for example, an atom or a molecule. The excited state is one of multiple possible energy states of the substance, having a higher energy than that of the ground state. The ground state of the substance is the energy state thereof having the lowest energy.

In the fields such as materials development and pharmaceutical development, analyzing the lowest energy state (ground state) is crucial for understanding of properties of a substance. Furthermore, analyzing the second or third lowest energy states (excited states) is necessary to determine whether a substance reacts or how readily a reaction proceeds.

Conventionally, SSVQE is sometimes used to calculate the energy of a substance's excited state. SSVQE is a method that minimizes a single objective function such as "lowest energy+w₂×second lowest energy+w₃×third lowest energy+ ...". Here, w (e.g., w₂, w₃) are weight coefficients for each energy. For more information on SSVQE, see, for example, Reference 1 below.

Reference 1: Subspace-search variational quantum eigensolver for excited states, Ken M. Nakanishi, Kosuke Mitari, and Keisuke Fujii, Phys. Rev. Research 1, 033062 Published 30 October 2019

In SSVQE, setting the weight coefficients for each energy to appropriate values is crucial for obtaining an optimal minimum solution. However, SSVQE has a problem in that setting these weight coefficients is difficult. For example, it is unclear whether there are settings for w₂, w₃, ... that can calculate the energies as expected. Therefore, it is inherently challenging to set the weights for each energy appropriately. When the weight coefficients cannot be set appropriately, it is impossible to accurately determine each energy.

Therefore, this embodiment describes an energy calculating method that enables the calculation of excited state energies without using weight coefficients for each energy (e.g., w₂, w₃), unlike existing SSVQE. The following describes an example of processing by the information processing device 100.

The information processing device 100 calculates respective states based on the values of parameters representing respective states of a substance using quantum chemical calculations. The information processing device 100 then calculates the energy of respective states based on the calculated states. Here, the substance is the target material for analysis, such as a candidate material or chemical.

Each state of the substance is one of multiple possible energy states of the substance. The states include the ground state and one or more excited states with a higher energy than that of the ground state. The states are, for example, mutually independent (orthogonal). The number of states is an integer of two or more and can be arbitrarily specified. For example, when calculating up to the third lowest energy (the energy of the second excited state), the number of states is specified as "3".

The parameters used to represent respective states may be one or more. For example, the parameters may be angling parameters used in the quantum circuits representing respective states. The angling parameters may be the angles of rotation around each axis (such as the X-axis, Y-axis, Z-axis, etc.). Quantum chemistry calculations are a method for analyzing the structure and properties of atoms and molecules from electron states thereof. Quantum chemistry calculations are performed, for example, by a quantum computer or quantum simulation.

In the example depicted in Fig. 1, the number of states of the substance is "3", and respective states are designated as "State S1, S2, and S3". The state S1 corresponds to the ground state. The state S2 corresponds to the first excited state. The state S3 corresponds to the second excited state. Furthermore, the values of the parameters used to represent respective states S1 to S3 are designated as "parameter values p#".

In this case, quantum chemical calculations determine respective states S1, S2, and S3 based on parameter values p#. Here, the state S1 calculated based on parameter values p# is denoted as "state S1(#)". Furthermore, the state S2 calculated based on the parameter values p# is denoted as "state S2(#)". Similarly, the state S3 calculated based on the parameter values p# is denoted as "state S3(#)".

Then, based on the calculated states S1(#), S2(#), and S3(#), the energies E1(#), E2(#), and E3(#) of the respective states S1(#), S2(#), and S3(#) are calculated. Energy E1(#) corresponds to the ground state energy. Energy E2(#) corresponds to the first excited state energy. Energy E3(#) corresponds to the second excited state energy.

The information processing device 100 repeatedly calculates the energies of respective states by repeatedly changing the values of the parameters representing respective states. In this case, the information processing device 100 performs the following processes (1-1) and (1-2).

(1-1) The information processing device 100 assigns (sets) ranks (priorities) to sets (groups) of calculated energies of respective states so that relatively higher ranks are assigned to sets corresponding to points relatively closer to the origin of a coordinate system including axes representing the energies of respective states. The origin is the point where all axes (coordinate axes) of the coordinate system intersect. The point corresponding to the set of energies of respective states means the point whose coordinates are represented by the energies of respective states. However, negative values may be calculated for the energies of the respective states. Therefore, the origin is not set for a case where the energy is zero, but is set to a value smaller than all calculated energy values.

For example, when the number of states is "3", the coordinate system becomes an orthogonal coordinate system including an axis representing the energy of the ground state (state S1), an axis representing the energy of the first excited state (state S2), and an axis representing the energy of the second excited state (state S3). The rank corresponds to an evaluation of each set of energies of the respective states. For example, a higher rank indicates that the energies are calculated as expected, signifying higher energy calculation accuracy. A higher rank indicates a better rank. A lower rank indicates a worse rank.

Here, a lower energy corresponds to a more stable state and can be considered the actual state of the substance. Furthermore, in the coordinate system including the axes representing the energy of each state, points relatively closer to the origin indicate a relatively lower overall energy. Therefore, the information processing device 100 assigns a relatively higher rank (evaluation) to points relatively closer to the origin in the coordinate system including the axes representing the energy of each state. The points correspond to the calculated sets of energies of respective states.

Here, a relatively lower numerical value of the rank indicates a relatively higher rank (evaluation). For example, a relatively higher rank indicates a relatively smaller rank value. A relatively lower rank indicates a relatively larger rank value. Being close to the origin indicates a small rank value. Being far from the origin indicates a large rank value. A high evaluation indicates a smaller rank value. A low evaluation indicates a large rank value. However, the rank may also be defined such that a relatively larger numerical value indicates a relatively higher rank (evaluation).

The rank assigned to the initial (first) energies among energies (sets of energies of respective states) which are repeatedly calculated while repeatedly changing the values of the parameters can be arbitrarily set. For example, the rank of the initial (first) energies set at "0".

In this case, when a point corresponding to the second energies (set of energies of respective states) is closer to the origin than a point corresponding to the first energies, the information processing device 100 assigns (set) rank "-1" to the second energies, for example.

On the other hand, when a point corresponding to the second energies is farther from the origin than the point corresponding to the first energies, the information processing device 100 assigns rank "+1" to the second energies, for example. Furthermore, when the distance of the point corresponding to the second energies from the origin is the same as that of the point corresponding to the first energy, the information processing device 100 may assign the rank "0" to the second energy.

In the example depicted in Fig. 1, it is assumed that rank R1 is assigned to the set (group) of energies E1(#), E2(#), and E3(#) of respective states S1(#), S2(#), and S3(#).

(1-2) The information processing device 100 changes the values of the parameters based on the assigned rank and the values of the parameters corresponding to that rank, so that the rank of the set of energies of respective states becomes higher. The "values of the parameters corresponding to that rank" means the values of the parameters had been used when calculating the set of energies of respective states to which the rank was assigned.

Specifically, for example, the information processing device 100 changes the values of the parameters by searching within preset ranges for the parameter values that will result in a higher rank for the next to be calculated set of energies of respective states, using an optimization algorithm. The parameter ranges can be set arbitrarily.

For example, the parameters may include angle parameters for rotation around each axis (X-axis, Y-axis, Z-axis, etc.). In this case, the ranges of the respective angle parameters for respective axes may be set at, for example, 0 degrees or more but less than 360 degrees, respectively. The initial values of the parameters may be set arbitrarily, for example. Any existing optimization algorithm may be used.

In the example depicted in Fig. 1, the values of the parameters are changed based on the rank R1 assigned to the set of energies E1(#), E2(#), and E3(#) of respective states S1(#), S2(#), and S3(#), and the parameter values p(#) corresponding to the ranking R1. Here, assume that the values of the parameters are changed to "parameter values p$".

In this case, the information processing device 100 calculates respective states S1, S2, and S3 based on the changed parameter values p$. Here, the state S1 calculated based on the parameter values p$ is denoted as "state S1($)". Furthermore, the state S2 calculated based on the parameter values p$ is denoted as "state S2($)". Similarly, the state S3 calculated based on the parameter values p$ is denoted as "state S3($)".

Then, the information processing device 100 calculates the energies E₁($), E₂($), and E₃($) of respective states S₁($), S₂($), and S₃($) based on the calculated respective states S₁($), S₂($), and S₃($). Energy E₁($) corresponds to the ground state energy. Energy E₂($) corresponds to the first excited state energy. Energy E₃($) corresponds to the second excited state energy.

Subsequently, in a similar manner, a rank R2 is assigned to the set of energies E1($), E2($), and E3($) of respective states S1($), S2($), and S3($). Based on the rank R2 and the parameter values p($) corresponding to the rank R2, the values of the parameters are further changed.

For example, the information processing device 100 repeats the energy calculation a predetermined number of times. Energy calculation is a series of processes that calculates the energies of respective states (e.g., states S1, S2, and S3) while repeatedly changing the values of the parameters representing (describing) respective states. The predetermined number is the upper limit on the number of iterations of the energy calculation and can be set arbitrarily.

Furthermore, the information processing device 100 outputs the calculated energies of respective states. Specifically, for example, the information processing device 100 may output the set of the calculated energies of respective states and a rank assigned to that set, in correspondence. Furthermore, after the information processing device 100 calculates sets of energies of respective states (e.g., states S1, S2, and S3) by repeatedly changing the values of the parameters, the information processing device 100 may output the set of energies of respective states to which the highest rank (e.g., rank R1, R2) is assigned.

As described, the information processing device 100 can calculate the energies of excited states of a substance. For example, by repeatedly changing the values of the parameters so as to increase the rank, the information processing device 100 can optimize the energies of respective states so as to be lower overall, thereby efficiently and accurately determining the energies of the excited states. Furthermore, since the information processing device 100 needs not set weight coefficients for respective energies different from the SSVQE, analysis of material properties is facilitated and user convenience is enhanced.

Here, while the functions of the information processing device 100 have been described as being implemented by a single computer, configuration is not limited hereto. For example, the functions of the information processing device 100 may be implemented through the collaboration of multiple computers. For instance, the functions of the information processing device 100 may be implemented by multiple computers in a cloud environment.

### (Example of an Information Processing System 200)

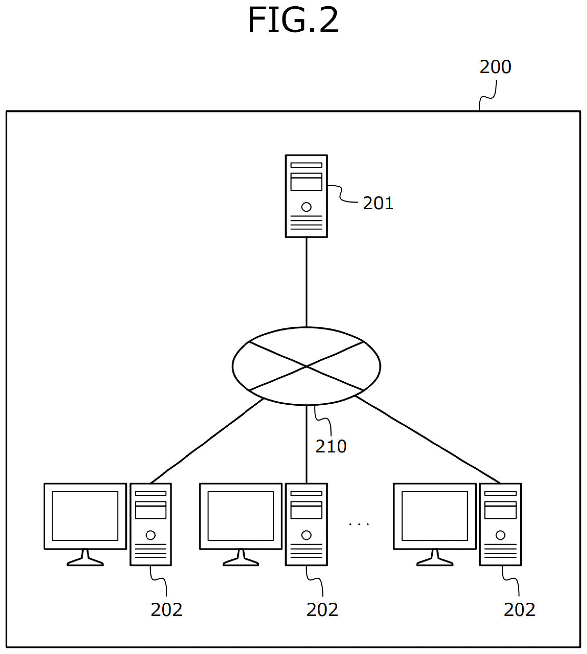

Next, with reference to Fig. 2, an example of an information processing system 200 to which the information processing device 100 depicted in Fig. 1 is applied, will be described. Here, an example is described where the information processing device 100 depicted in Fig. 1 is applied to the excitation energy calculating device 201 in the information processing system 200.

Fig. 2 is an explanatory diagram depicting an example of the information processing system 200. In Fig. 2, the information processing system 200 includes an excitation energy calculating device 201 and client devices 202.

In the information processing system 200, the excitation energy calculating device 201 and the client device 202 are coupled via a wired or wireless network 210. The network 210 may be, for example, a LAN (Local Area Network), WAN (Wide Area Network), or the Internet.

The excitation energy calculating device 201 is a computer that calculates the energies of the excited states of a substance. The excitation energy calculating device 201 receives a processing request from the client device 202, requesting the calculation of the energies of the excited state of a substance-under-analysis.

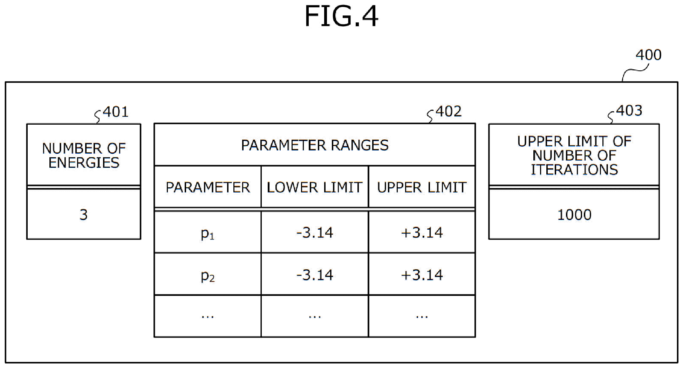

The processing request includes, for example, information identifying the substance-under-analysis. The processing request may also include, for example, energy calculation setting information. The energy calculation setting information includes various settings related to energy calculation. An example of the energy calculation setting information will be described later with reference to Fig. 4.

The excitation energy calculating device 201 calculates the energies of the excited states of the substance-under-analysis in response to the processing request. The excitation energy calculating device 201 then transmits the calculated energies of the excited states of the substance-under-analysis to the client devices 202. The excitation energy calculating device 201 is, for example, a server or a PC.

Each of the client devices 202 is a computer used by a user. The user is, for example, an analyst who analyzes properties of the substance-under-analysis. Based on manipulation input of the analyst, the client device 202 generates a processing request requesting calculation of the energies of the excited states of the substance-under-analysis and transmits the request to the excitation energy calculating device 201.

The client device 202 receives the energies of excited states of the substance-under-analysis from the excitation energy calculating device 201. The client device 202 outputs the energies of the excited states of the substance-under-analysis for the analyst to reference. The client device 202 is, for example, a PC, tablet device, or smartphone.

The description here pertains to a case where the excitation energy calculating device 201 is a computer different from the client device 202, but this is not limited thereto. For example, the excitation energy calculating device 201 may have the functionality of a client device 202 and may also operate as a client device 202.

### (Example of Hardware Configuration of Excitation Energy Calculating Device 201)

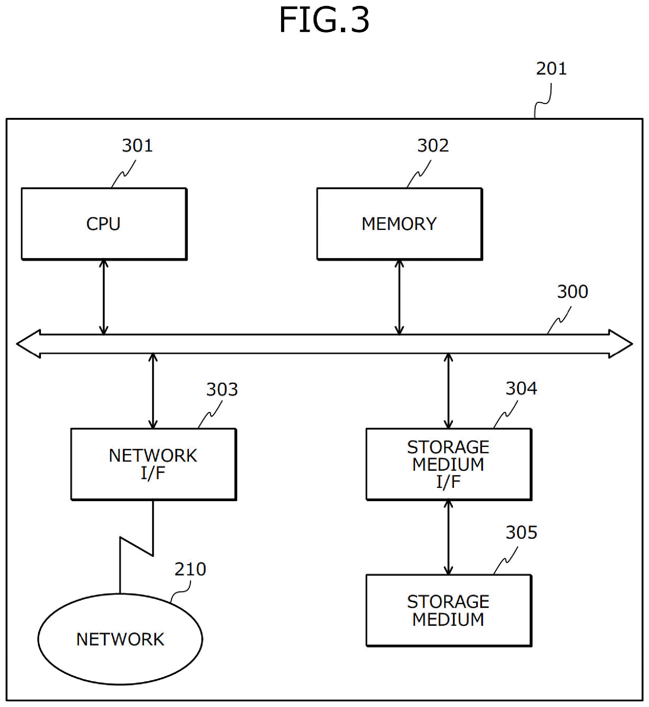

Next, an example of a hardware configuration of the excitation energy calculating device 201 is described with reference to Fig. 3.

Fig. 3 is a block diagram of an example of a hardware configuration of the excitation energy calculating device 201. In Fig. 3, of the excitation energy calculating device 201 has a central processing unit (CPU) 301, a memory 302, a network interface (I/F) 303, a recording medium I/F 304, and a recording medium 305. Further, the components are connected to each other by a bus 300.

Here, the CPU 301 governs overall control of the information processing device 100. The memory 302, for example, includes a read-only memory (ROM), a random access memory (RAM), and a flash-ROM. In particular, for example, the flash-ROM and/or ROM stores therein various programs and the RAM is used as a work area of the CPU 301. Programs stored to the memory 302 are loaded onto the CPU 301, whereby encoded processes are executed by the CPU 301.

The network I/F 303 is coupled to the network 210 via a communications line and is coupled to other computers through the network 210. Further, the network I/F 303 administers an internal interface with the network 210 and controls the input and output of data with respect to the other computers. The network I/F 303, for example, is a modem, a LAN adapter, or the like.

The recording medium I/F 304 controls the reading and writing of data with respect to the recording medium 305 under the control of the CPU 301. The recording medium I/F 304 is, for example, a disk drive, a solid-state drive (SSD), a universal serial bus (USB) port, or the like. The recording medium 305 is a nonvolatile memory storing data written thereto under the control of the recording medium I/F 304. The recording medium 305 is, for example, a disk, a semiconductor memory, a USB memory, or the like. The recording medium 305 may be removable from the excitation energy calculating device 201.

In addition to the components above, the excitation energy calculating device 201 may include, for example, a keyboard, a mouse, a display, a printer, a scanner, a microphone, a speaker, etc. Further, the excitation energy calculating device 201 may further have the recording medium I/F 304 and/or the recording medium 305 in plural. The information processing device 100 may omit the recording medium I/F 304 and/or the recording medium 305.

### (Example of Hardware Configuration for Client Device 202)

An example of an example of a hardware configuration of each client device 202 is basically a same as the example of the hardware configuration example of the excitation energy calculating device 201 depicted in Fig. 3 and thus, the description is omitted. However, the client devices 202 include, in addition to the components depicted in Fig. 3, for example, a keyboard, a mouse, and a display.

### (Example of Energy Calculation Setting Information)

Next, an example of energy calculation setting information is described with reference to Fig. 4.

Fig. 4 is an explanatory diagram depicting an example of energy calculation setting information. In Fig. 4, the energy calculation setting information 400 includes a number of energies 401, parameter ranges 402, and an upper limit 403 of the number of iterations of calculation.

The number of energies 401 indicates the number of energies to be calculated. The number of energies 401 corresponds to the number of states of the substance. For example, if the number of energies 401 is "3", the energies of the ground state, the first excited state, and the second excited state are calculated.

The parameter ranges 402 indicates the ranges of the parameters (e.g., parameters p₁, p₂) used to represent respective states of the substance-under-analysis. The parameter ranges 402 include the lower limit and upper limit values for each parameter. For example, parameter ranges 402 include a lower limit value "-3.14" and an upper limit value "+3.14" for parameter p₁. Parameter ranges 402 also include a lower limit value "-3.14" and an upper limit value "+3.14" for parameter p₂.

The upper limit 403 of the number of iterations of calculation indicates the maximum number of times the energy calculation is repeated. For example, when the upper limit 403 of the number of iterations of calculation is "1000", the process of calculating the energies of respective states of the substance-under-analysis (energy calculation) is repeated 1000 times while repeatedly changing the values of the parameters (e.g., parameters p₁, p₂).

### (Example of Functional Configuration of Excitation Energy Calculation Device 201)

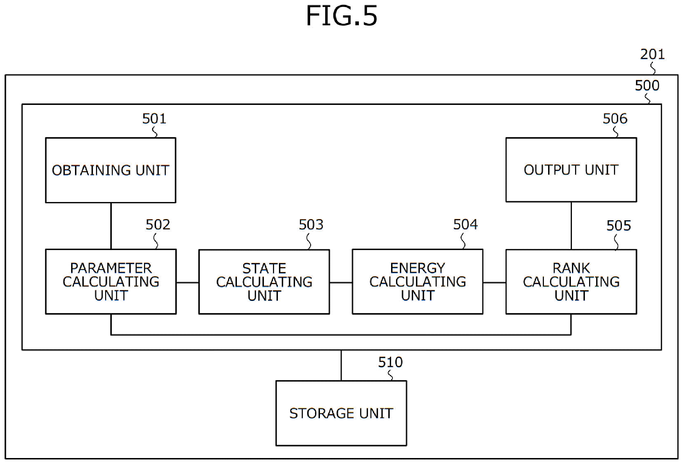

Next, with reference to Fig. 5, an example of the functional configuration of the excitation energy calculating device 201 is described.

Fig. 5 is a block diagram depicting an example of the functional configuration of the excitation energy calculating device 201. In Fig. 5, the excitation energy calculating device 201 includes an obtaining unit 501, a parameter calculating unit 502, a state calculating unit 503, an energy calculating unit 504, a rank calculating unit 505, an output unit 506, and a storage unit 510.

The obtaining unit 501 to the output unit 506 constitute a controller 500. Specifically, the functions thereof are realized, for example, by causing the CPU 301 to execute a program stored in a storage area such as the memory 302 or storage medium 305 depicted in Fig. 3, or via the network I/F 303. The processing results of each functional unit are stored to a storage area, such as the memory 302 or the storage medium 305 depicted in Fig. 3.

The storage unit 510 is implemented, for example, by storage areas such as the memory 302 and storage media 305 depicted in Fig. 3. While the following description pertains to a case where the storage unit 510 is included in the excitation energy calculating device 201, configuration is not limited hereto. For example, the storage unit 510 may be included in a device different from the excitation energy calculating device 201 and the contents stored in the storage unit 510 may be accessible for reference by the excitation energy calculating device 201. The storage unit 510 stores various information referenced or updated during the processing of each functional unit.

The obtaining unit 501 obtains various information used in the processes by the functional units. The obtaining unit 501 stores the various obtained information to the storage unit 510 and outputs the information to the functional units. Furthermore, the obtaining unit 501 may output various information stored in the storage unit 510 to the functional units. The obtaining unit 501 obtains various information, for example, based on user manipulation input. The obtaining unit 501 may, for example, receive various information from a device different from the excitation energy calculating device 201.

The obtaining unit 501 obtains, for example, a processing request requesting the calculation of energies of the excitation states of a substance-under-analysis. Specifically, the obtaining unit 501 obtains the processing request by receiving the processing request from another computer. The other computer is, for example, one of the client devices 202 depicted in Fig. 2. The obtaining unit 501 may also obtain the processing request by accepting input of the processing request based on user manipulation input.

The obtaining unit 501 obtains information identifying the substance-under-analysis. Specifically, the obtaining unit 501 obtains the information identifying the substance-under-analysis by receiving the information from another computer. The other computer is, for example, one of the client devices 202. Furthermore, the obtaining unit 501 may obtain information identifying the substance-under-analysis by accepting input of such information based on user manipulation input. Additionally, when the information identifying the substance-under-analysis is included in the processing request, the obtaining unit 501 may obtain the substance-under-analysis by extracting the information identifying the substance-under-analysis from the processing request.

The obtaining unit 501 obtains, for example, energy calculation setting information. Here, the energy calculation setting information includes various settings concerning the energy calculation of the substance-under-analysis. The energy calculation setting information includes, for example, the number of energies to be calculated, the ranges of parameters for representing each state of the substance-under-analysis, and the upper limit of the number of iterations.

Specifically, the obtaining unit 501 obtains the energy calculation setting information 400 depicted in Fig. 4 by receiving the energy calculation setting information 400 from another computer. The other computer is, for example, one of the client devices 202. Furthermore, the obtaining unit 501 may obtain the energy calculation setting information 400 by accepting input of the energy calculation setting information 400 based on user manipulation input. Additionally, the obtaining unit 501 may obtain the energy calculation setting information 400 by extracting the energy calculation setting information 400 from the processing request in a case where the energy calculation setting information 400 is included in the processing request.

The obtaining unit 501 may receive a start trigger to initiate processing by any functional unit. The start trigger may be, for example, a predetermined user manipulation input. The start trigger may also be, for example, the reception of predetermined information from another computer. The start trigger may also be, for example, the output of predetermined information by any functional unit. Specifically, for example, the obtaining unit 501 may regarding obtaining the processing request as a start trigger to initiate processes by the parameter calculating unit 502 to the output unit 506.

In the following description, multiple states including the ground state and excited states (one or more excited states) of the substance-under-analysis may be denoted as "State₁ to State_{Ns}". Ns represents the number of states, corresponding to, for example, the number of energies to be calculated. For example, the state State₁ represents the ground state. The state State₂ represents the first excited state. The state State₃ represents the second excited state.

Additionally, any state among State₁ to State_{Ns} may be denoted as "Stateⱼ" (j=1, 2, ..., Ns). Furthermore, the energy of each state Stateⱼ may be denoted as "Energyⱼ". Additionally, the parameters used to represent each state Stateⱼ may be denoted as "parameters p".

The parameter calculating unit 502 calculates the values of the parameters p. The parameters p includes one or more parameters for representing each state Stateⱼ. Specifically, for example, the parameter calculating unit 502 calculates the initial values of the parameters p in the initial state. Here, the initial state refers to the state before the first (initial) energy calculation is performed, where the energies Energyⱼ of respective states Stateⱼ have not yet been calculated, and the rank to each set of states Stateⱼ and the corresponding energies Energyⱼ thereof has not yet been assigned.

The initial values of parameters p may be preset or randomly selected. In this case, the parameter calculating unit 502 calculates the values of parameters p using either preset values of parameters p or randomly selected values of parameters p. Furthermore, the initial values of parameters p may be calculated based on the optimization algorithm which is used when repeatedly changing the values of parameters p, for example.

The specific processing details for calculating (changing) the values of parameters p in energy calculations performed after the second time will be described later.

The state calculating unit 503 calculates each state Stateⱼ based on the values of parameters p using quantum chemical calculations. Specifically, for example, the state calculating unit 503 performs quantum chemical calculations using a quantum computer or quantum simulation. The state calculating unit 503 then provides the values of parameters p to the quantum circuit U(p) representing each state Stateⱼ to calculate each state Stateⱼ.

Quantum simulation performs quantum computation by reproducing quantum states on conventional computers and calculating interactions between qubits, among other operations. A quantum computer is a computer that utilizes the principles of quantum mechanics. A quantum circuit is a computational model combining quantum gates that control the quantum states of qubits. Quantum circuits are created, for example, according to the substance-under-analysis.

When performing quantum chemistry calculations using a quantum computer, the quantum computer may include the excitation energy calculating device 201, or the quantum computer may be an external device connected to the excitation energy calculating device 201 via the network 210 depicted in Fig. 2.

Here, Np denotes the number of parameters p, and the Np parameters p are denoted as "p₁, p₂, ..., p_{Np}". Furthermore, Nv=2^{Ns} is assumed. Ns is the number of states Stateⱼ. Ns is determined, for example, from the number of energies 401 included in the energy calculation setting information 400. A state Stateⱼ is represented, for example, by a complex number. A complex number has a real part and an imaginary part. Therefore, for each state Stateⱼ, there are two elements including the real part and the imaginary part. Nv corresponds to the number of all combinations that are to be computed as state Stateⱼ. Furthermore, Tentativeⱼ(I) is a tentative vector of the complex number representing state Stateⱼ. Equation (I) below represents an operation for Tentativeⱼ(I) where the real part remains unchanged and the imaginary part is negated. Furthermore, Equations (2) and (3) below shall hold for Tentativeⱼ(I). Here, "j=1, 2, ..., Ns", "k=1, 2, ..., Ns", and "I=1, 2, ..., Nv". 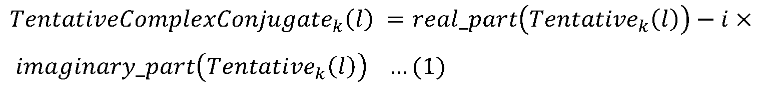
TentativeComplexConjugatek(l) = real_part(Tentativek(l)) - i × imaginary_part(Tentativek(l)) ...
${\sum }_{l = 1}^{{N}_{v}} {TentativeComplexConjugate}_{k} \left(l\right) \times {Tentative}_{j} \left(l\right) = 0 ⋯ j \neq k$
${\sum }_{l = 1}^{{N}_{v}} {TentativeComplexConjugate}_{k} \left(l\right) \times {Tentative}_{j} \left(l\right) = 1 ⋯ j = k$

In this case, the state calculating unit 503 can calculate each state Stateⱼ using, for example, the following equation (4). Here, CalculateStateₚ₁, ₚ₂,...,_{pNp} is a function containing parameters p₁, p₂, ..., p_{Np}, and is, for example, an array determined by the parameters p₁, p₂,..., p_{Np}. Furthermore, "m=1, 2, ..., Nv" and "n=1, 2, ..., Nv" are assumed.
${State}_{j} \left(m\right) = {\sum }_{n = 1}^{{N}_{v}} {CalclateState}_{{p}_{1} , {p}_{2} , ⋯ , {p}_{{N}_{p}}} \left(m, n\right) \times {Tentative}_{j} \left(n\right)$

Furthermore, any existing technique may be used for the method of calculating each state Stateⱼ based on the values of the parameters p by quantum chemical calculation.

The energy calculating unit 504 calculates the energies Energyⱼ of the respective states Stateⱼ based on the calculated state Stateⱼ. Specifically, for example, the energy calculating unit 504 can calculate the energy Energyⱼ of each state Stateⱼ using the following Equations (5) and (6). Equation (5) represents an operation of leaving the real part unchanged and attaching a negative sign to the imaginary part for Stateⱼ. StateComplexConjugateⱼ(I) is a matrix with 1 row and I (L) columns. CalculateEnergy(l, m) is a I row × m columns matrix. Stateⱼ(m) is an m rows × 1 column matrix.
$\begin{matrix}{StateComplexConjugate}_{j} \left(l\right) = real _ part \left({State}_{j}\left(l\right)\right) - i \times \\ imaginary _ part \left({State}_{j}\left(l\right)\right)\end{matrix}$
$\begin{matrix}{StateComplexConjugate}_{j} \left(l\right) \\ {Energy}_{j} = {\sum }_{l = 1}^{{N}_{v}} {\sum }_{m = 1}^{{N}_{v}} \begin{matrix}\times CalclateEnergy \left(l, m\right) \\ \times {State}_{j} \left(m\right)\end{matrix}\end{matrix}$

Furthermore, any existing technique may be used to calculate the energies Energyⱼ of the respective states Stateⱼ based on the states Stateⱼ.

The rank calculating unit 505 assigns a rank to each set of calculated energies Energyⱼ of the respective states Stateⱼ. Specifically, for example, the rank calculating unit 505 assigns the rank so that, in a coordinate system including axes representing the energies Energyⱼ of the respective states Stateⱼ, the sets that correspond to points relatively closer to the origin are ranked relatively higher. The points respectively correspond to the calculated sets of energies Energyⱼ of states Stateⱼ.

In the following description, the coordinate system including the axes representing energies Energyⱼ of respective states Stateⱼ may be denoted as "coordinate system CS (Coordinate System)". The coordinate system CS is, for example, an orthogonal coordinate system. The origin of the coordinate system CS may be denoted as "origin O".

For the energies Energyⱼ of the respective states Stateⱼ, a negative value may sometimes be calculated. Therefore, the origin O is not set for the case where the energies are 0, but is set to be smaller than all calculated energy values. Furthermore, the rank indicates that a relatively smaller numerical value is a relatively higher rank (evaluation).

Whether a point corresponding to a set of energies Energyⱼ of the respective states Stateⱼ is close to the origin O is determined by a distance thereof from the origin O. The distance from the origin O for a given set may be represented by the number of other sets where all energies Energyⱼ of the respective states Stateⱼ are lower than those of the given set. In this case, a relatively larger number of other sets indicates a relatively greater distance from the origin O.

To explain in more detail, for example, the rank calculating unit 505 assigns a predetermined rank to the first (initial) set of energies Energyⱼ. The rank to be assigned to the first (initial) set of energies Energyⱼ is set to, for example, "0".

The first set of energies Energyⱼ is a first calculated set of energies Energyⱼ among sets of energies Energyⱼ calculated while repeatedly changing parameters p.

Furthermore, the second set of energies Energyⱼ is assumed to be closer to the origin O than the first set of energies Energyⱼ. In this case, the rank calculating unit 505 assigns "the rank of the first set minus one (the rank of the first set - 1)" to the second set of energies Energyⱼ".

Furthermore, the second set of energies Energyⱼ is assumed to have the same distance from the origin O as that of the first set of energies Energyⱼ. In this case, the rank calculating unit 505 assigns "the rank of the first (initial) set" to the second set of energies Energyⱼ.

Furthermore, the second set of energies Energyⱼ is assumed to be farther from the origin O than is the first set of energies Energyⱼ. In this case, the rank calculating unit 505 assigns "the rank of the first set plus one (the rank of the first set + 1)" to the second set of energies Energyⱼ.

Furthermore, the third and subsequent sets of energies Energyⱼ are assumed to be closer to the origin O than is the set of energies Energyⱼ having the highest rank. In this case, the rank calculating unit 505 sets "the highest rank minus one (the highest rank - 1)" for the third and subsequent sets of energies Energyⱼ.

Furthermore, the third and subsequent sets of energies Energyⱼ are assumed to have the same distance from the origin O as the set of energies Energyⱼ having the highest rank. In this case, the rank calculating unit 505 assigns "the highest rank" to the sets of energies Energyⱼ.

Furthermore, the distances from the origin O to the third and subsequent sets of energies Energyⱼ are assumed to lie between the distance for the k-th (k: natural number) highest-ranked set of energies Energyⱼ and the distance for the (k+1)-th highest-ranked set of energies Energyⱼ. In this case, the rank calculating unit 505 assigns "0.5 × (k-th highest rank + (k+1)-th highest rank)" to the third and subsequent sets of energies Energyⱼ.

Here, using the first (first execution) to third iteration (execution) of calculation as examples, examples of assigning the rank to each set of energies Energyⱼ in each iteration are described.

First, the rank calculating unit 505 assigns the rank "0" to the first (first execution) set of energies Energyⱼ.

Next, the rank calculating unit 505 assigns the rank "-1" to the set of energies Energyⱼ of the respective states Stateⱼ newly calculated in the second calculation iteration when the calculated set is closer to the origin O than is the initial (first) set calculated in the first iteration. On the other hand, when the newly calculated set is farther from the origin O than is the initial (first) set calculated in the first iteration, the rank calculating unit 505 assigns the rank "+1" to the newly calculated set. Furthermore, when the calculated distance from the origin O is the same as that of the initial (first) set, the rank calculating unit 505 assigns the rank "0" to the newly calculated set.

Next, described is the rank of the set of energies Energyⱼ in the third iteration. Here, it is assumed that rank "0" was assigned to the first set of energies Energyⱼ calculated in the first iteration, and rank "1" was assigned to set of energies Energyⱼ of the second Stateⱼ calculated in the second iteration.

Further, the third set of energies Energyⱼ calculated in the third iteration is assumed to be closer to the origin O than is the first set of energies Energyⱼ having the highest rank. In this case, the rank calculating unit 505 assigns rank "-1" to the third and subsequent sets of energies Energyⱼ.

Furthermore, the third set of energies Energyⱼ is assumed to have the same distance from the origin O as that of the set of energies Energyⱼ having the highest rank (the first set). In this case, the rank calculating unit 505 assigns the rank "0" to the third and subsequent sets of energies Energyⱼ.

Furthermore, the distances from the origin O to the third sets of energies Energyⱼ are assumed to be between the distance of the first-ranked (initial) set of energies Energyⱼ and the distance of the second-ranked set of energies Energyⱼ. In this case, the rank calculating unit 505 assigns the rank "0.5" to the third set of energies Energyⱼ.

Furthermore, the third set of energies Energyⱼ are assumed to be farther from the origin O than is the lowest-ranked (second) set of energies Energyⱼ. In this case, the rank calculating unit 505 assigns the rank "2" to the third and subsequent sets of energies Energyⱼ.

In the description above, when assigning a rank to a set of energies Energyⱼ of the respective states Stateⱼ, while an example is described where, when the distance from the origin O of the coordinate system CS is the same as those of other sets, the same rank is assigned to the other sets. However, configuration is not limited hereto. For example, another set whose distance from the origin O is the same as that of the calculated set of energies Energyⱼ of the respective states Stateⱼ is assumed to be present.

In this case, the rank calculating unit 505 may assign to a set of energies Energyⱼ of the calculated states Stateⱼ, a rank that is higher than that of other sets when the energy of a lower state among State₁ to State_{Ns} of the calculated states Stateⱼ is lower than the energy of the other sets. Furthermore, the rank calculating unit 505 may assign a rank lower than those of other sets when the energy of the lower state is higher than that of other sets.

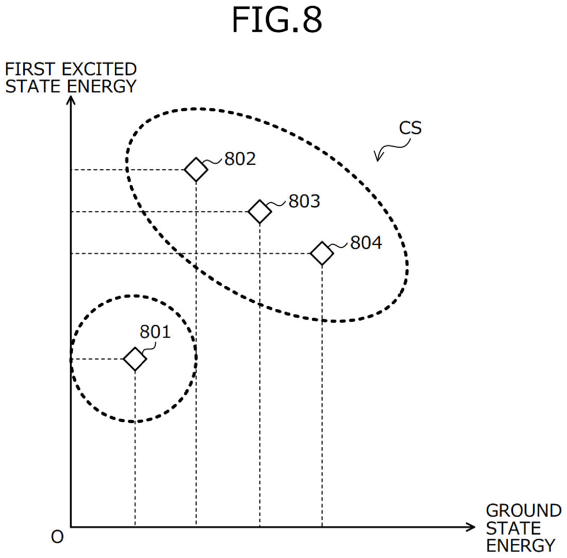

An example of assigning the rank to the set of energies Energyⱼ of respective states Stateⱼ will be described later with reference to Fig. 8.

The rank calculating unit 505 stores the assigned rank in correspondence with the values of parameters p corresponding to the rank. The values of parameters p corresponding to the rank are the values of parameters p used when calculating the set of energies Energyⱼ of respective states Stateⱼ to which the rank was assigned. At this time, the rank calculating unit 505 may further store the calculated states Stateⱼ and energies Energyⱼ of each state Stateⱼ in correspondence with the assigned rank and the values of the parameters p corresponding to the rank.

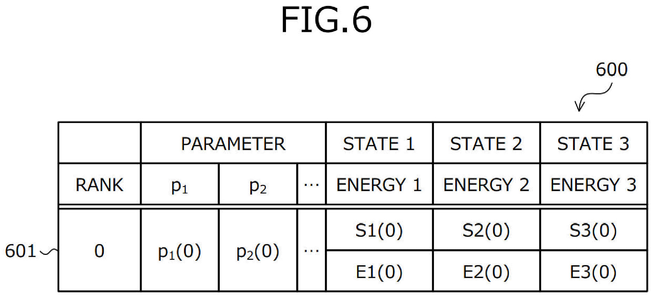

Specifically, for example, the rank calculating unit 505 stores the calculated states Stateⱼ and the energies Energyⱼ of the respective states Stateⱼ in a calculation result table 600, such as that depicted in Fig. 6, associating the respective calculated states Stateⱼ and the energies Energyⱼ with the assigned rank and the values of parameters p corresponding to the rank.

Here, with reference to Fig. 6, the stored contents of the calculation result table 600 are explained. Here, Ns (the number of states Stateⱼ) is set to "Ns=3" based on the number of energies 401 included in the energy calculation setting information 400.

Fig. 6 is an explanatory diagram depicting an example of the stored contents of the calculation result table 600. In Fig. 6, the calculation result table 600 has fields for the rank, the parameters (p₁, p₂, ...), the states (state 1, state 2, and state 3), and the energies (energy 1, energy 2, and energy 3). By setting information in each field, a calculation result (e.g., calculation result 601) is stored as a record.

Here, the rank is the rank of the set of energies Energyⱼ of the respective states Stateⱼ of the substance. A smaller rank value indicates a higher evaluation. The parameters are the values p (p₁, p₂, ...) describing the respective states Stateⱼ. For explanatory purposes, the parameter p₁ for rank k is denoted as "p₁(k)", and the parameter p₂ for rank k is denoted as "p₂(k)" (k is an integer).

The states (state 1, state 2, and state 3) represent the states Stateⱼ of the substance. State 1 represents the state State₁, corresponding to the ground state of the substance. State 2 represents the state State₂, corresponding to the first excited state of the substance. State 3 represents the state State₃, corresponding to the second excited state of the substance. For explanatory convenience, state 1 of rank "k" is denoted as "S1(k)", state 2 of rank "k" as "S2(k)", and state 3 of rank "k" as "S3(k)".

Energies (energy 1, energy 2, energy 3) represent the energies Energyⱼ of the respective states Stateⱼ of the substance. Energy 1 represents energy Energy₁ and corresponds to the ground state energy. Energy 2 represents Energy₂ and corresponds to the energy of the first excited state energy. Energy 3 represents energy Energy₃, corresponding to the second excited state energy. For explanatory convenience, the energy of state 1 with rank "k" is denoted as "E1(k)", the energy of state 2 with rank "k" is denoted as "E2(k)", and the energy of state 3 with rank "k" is denoted as "E3(k)".

In the example depicted in Fig. 6, the calculation result 601 is stored as a result of calculating the initial (first) set of energies Energyⱼ of respective states Stateⱼ. In the calculation result 601, since this is the initial (first) calculation, a rank of "0" is assigned to set of energies Energyⱼ of the respective states Stateⱼ.

Although not depicted, each calculation result stored in the calculation result table 600 may also include the distance from the origin O of the coordinate system CS corresponding to the point associated with the set of energies Energyⱼ of the respective states Stateⱼ.

Here, the description of Fig. 5 continues, the parameter calculating unit 502 changes the values of parameters p based on the assigned rank and the values of parameters p corresponding to that rank, such that the rank assigned to the set of energies Energyⱼ of the respective states Stateⱼ becomes higher. The values of the parameters p are changed, for example, during the second or subsequent energy calculations.

Specifically, for example, the parameter calculating unit 502 identifies the assigned rank and values of the corresponding parameters p by referring to the calculation result table 600 depicted in Fig. 6. Then, based on the identified rank and the corresponding values of parameters p, the parameter calculating unit 502 changes the values of parameters p using an optimization algorithm.

More specifically, for example, the parameter calculating unit 502 changes the values of parameters p by searching within preset search ranges for values of parameters p yielding a higher rank for the next set of energies Energyⱼ to be calculated of the respective states Stateⱼ. The search ranges are set respectively for the parameters p₁, p₂, ..., p_{Np}, for example. The search ranges are identified from the parameter ranges 402 included in the energy calculation setting information 400, for example.

Any existing optimization algorithm may be used. Examples of optimization algorithms include the Nelder-Mead method, Powell's conjugate direction method, BFGS (Broyden-Fletcher-Goldfarb-Shanno algorithm), SLSQP (Sequential Least Quadratic Programming) method, simulated annealing, genetic algorithms, differential evolution algorithms, and particle swarm optimization, etc.

When the values of parameters p are changed, the state calculating unit 503 calculates respective states Stateⱼ based on the changed values of parameters p using quantum chemical calculations. As described, the excitation energy calculating device 201 performs subsequent energy calculations while repeatedly changing the values of parameters p.

For example, the excitation energy calculating device 201 repeats the energy calculation a predetermined number of times while repeatedly changing the values of parameters p. The predetermined number is an upper limit of the number of iterations of the energy calculation and is determined, for example, from the upper limit 403 of the number of iterations of calculation included in the energy calculation setting information 400.

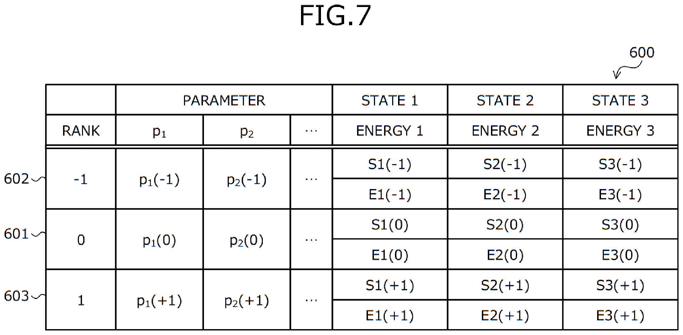

Here, with reference to Fig. 7, an example of updating the stored contents of the calculation result table 600 is described. Specifically, the stored contents of the calculation result table 600 after the second and third energy calculations have been performed are described.

Fig. 7 is an explanatory diagram depicting an example of updating the stored contents of the calculation result table 600. In Fig. 7, the calculation result table 600 stores calculation results 601 to 603. The calculation result 601 is the result stored as described with reference to Fig. 6 and represents the first (first time) set of energies Energyⱼ of the respective states Stateⱼ.

Calculation result 602 is stored as the result of calculating the set of energies Energyⱼ of the respective states Stateⱼ in the second iteration of calculation. In the calculation result 602, a higher rank "-1" is assigned to the set of energies Energyⱼ of the respective states Stateⱼ in the second iteration, as compared to the first iteration calculation.

Furthermore, calculation result 603 is the result stored after calculating the third set of energies Energyⱼ of the respective states Stateⱼ. In the calculation result 603, a lower rank "1" is assigned to the set of energies Energyⱼ of the respective states Stateⱼ in the third iteration, as compared to the first iteration calculation.

Here, the description of Fig. 5 continues. The output unit 506 outputs the calculated energies Energyⱼ of respective states Stateⱼ. The output formats of the output unit 506 include, for example, storage to the memory 302 or the storage medium 305, transmission to other computers via network I/F 303, display on a non-depicted display, and printing output to a non-depicted printer.

Specifically, for example, the output unit 506 outputs, among sets of energies Energyⱼ of respective states Stateⱼ calculated by repeatedly changing the values of parameters p, a set of energies Energyⱼ of respective states Stateⱼ having the highest rank.

To explain in more detail, for example, after the energy calculation is performed a predetermined number of times (upper limit 403 of number of iterations), the output unit 506 identifies the highest-ranked set of energies Energyⱼ of respective states Stateⱼ by referring to the calculation result table 600. Then, the output unit 506 outputs the identified set of energies Energyⱼ of respective states Stateⱼ as energy analysis results.

At this time, the output unit 506 may also identify the values of parameters p with the highest rank by referring to the calculation result table 600. The output unit 506 may then output the identified values of parameters p and the identified set of energies Energyⱼ of respective states Stateⱼ as energy analysis results.

The energy analysis results may include, for the best rank (highest rank), the values of parameters p, each state Stateⱼ, the energies Energyⱼ of the respective states Stateⱼ, and the rank. Furthermore, the energy analysis results may include, for all ranks, the values of parameters p, each state Stateⱼ, the energies Energyⱼ of the respective states Stateⱼ, and the rank.

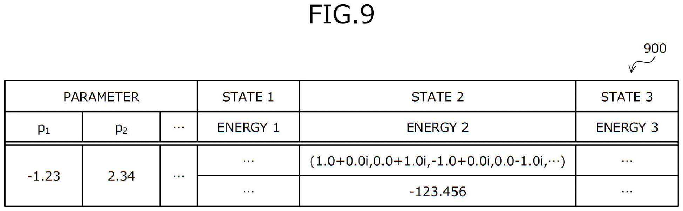

Specific examples of the energy analysis results will be described later with reference to Fig. 9.

The output unit 506 may also output only the energies Energyⱼ of a specific state Stateⱼ (e.g., a first excited state) from among the states State₁ to State_{Ns}. Furthermore, when there are multiple highest-ranked sets of energies Energyⱼ of respective states Stateⱼ, the output unit 506 may output any one of the highest-ranked sets, or may output all of the highest-ranked sets.

Furthermore, past energy calculation results may exist for the substance-under-analysis. In this case, the excitation energy calculating device 201 may utilize the past energy calculation results to optimize the values of parameters p.

For example, obtaining unit 501 obtains past calculation result data. Here, the past calculation result data represents the assigned rank and the values of parameters p corresponding to the rank, with respect to each of the sets of energies Energyⱼ of the respective states Stateⱼ calculated in the past. The rank is assigned to the set so that relatively higher ranks are assigned to the sets corresponding to points relatively closer to the origin O in the coordinate system CS. The past calculation result data may, for example, be created by randomly sampling values of the parameters p.

Specifically, for example, obtaining unit 501 obtains past calculation result data by receiving the data from another computer. The other computer is, for example, client device 202 depicted in Fig. 2. The obtaining unit 501 may also obtain past calculation result data by accepting input of past calculation result data based on user's manipulation input.

The data structure of the past calculation result data is similar to the calculation result table 600 depicted in Figs. 6 and 7, for example, and is therefore not depicted.

Furthermore, when past calculation result data is obtained, the rank calculating unit 505 may assign a rank to the calculated set of the energies Energyⱼ of the respective states Stateⱼ by referring to the past calculation result data, whereby the rank calculating unit 505 may assign the ranks such that relatively higher ranks are assigned to the sets corresponding to the points that are relatively closer to the origin O. In this case, the rank calculating unit 505 assigns a consistent rank to the calculated set of energies Energyⱼ of the respective states Stateⱼ, for example, so as not to change the rankings in the past calculation result data.

Furthermore, when past calculation result data is obtained, the parameter calculating unit 502 may change the values of parameters p so that the rank to be assigned to the set of energies Energyⱼ of the respective states Stateⱼ becomes higher, based on the assigned rank, the values of parameters p corresponding to the rank, and the past calculation result data.

Thus, the parameter calculating unit 502 optimizes the values of parameters p by considering the combination of the ranks assigned to each of the sets of energies Energyⱼ of the respective states Stateⱼ and the values of parameters p corresponding to the ranks calculated in the past.

Furthermore, during the initial (first) energy calculation, the parameter calculating unit 502 may calculate the values of parameters p such that the rank to be assigned to the set of energies Energyⱼ becomes higher, based solely on past calculation result data.

Furthermore, when the energies Energyⱼ of the respective states Stateⱼ are repeatedly calculated while repeatedly changing the values of parameters p, the highest rank may be included in past calculation result data. In this case, the output unit 506 outputs the highest-ranked set of energies Energyⱼ of each state Stateⱼ by referring to the past calculation result data.

The functional units (obtaining unit 501 to output unit 506) of the excitation energy calculating device 201 may be implemented, for example, by multiple computers in the information processing system 200 (e.g., the excitation energy calculating device 201 and the client devices 202). In this case, communication between functional units on different computers is performed, for example, by transmission and reception between the functional units via the network 210.

### (Example of Assigning Ranks)

Next, with reference to Fig. 8, an example of assigning a rank to a set of energies Energyⱼ of the respective states Stateⱼ is described.

Fig. 8 is an explanatory diagram depicting an example of assigning ranks. In Fig. 8, points 801 to 804 plotted in the coordinate system CS are depicted. The coordinate system CS is a coordinate system including axes representing the energies Energyⱼ of respective states Stateⱼ. However, in Fig. 8, only the axis representing the energy Energy₁ (ground state energy) of state State₁ and the axis representing the energy Energy₂ (first excited state energy) of state State₂, from among the axes representing the energies Energy₁ to Energy_{Ns} of states State₁ to State_{Ns}, are depicted.

Point 801 corresponds to the set of energies Energyⱼ of the respective states Stateⱼ calculated at the k-th iteration. Point 802 corresponds to the set of energies Energyⱼ of the respective states Stateⱼ calculated at the (k+1)-th iteration. Point 803 corresponds to the set of energies Energyⱼ of the respective states Stateⱼ calculated at the (k+2)-th iteration. Point 804 corresponds to the set of energies Energyⱼ of the respective states Stateⱼ calculated at the (k+3)-th iteration.

Here, among points 801 to 804, point 801 is the closest to the origin O. As an example, the respective distances from the origin O to points 801 to 804 are represented by the number of other points for which all of the energies Energyⱼ of the respective states Stateⱼ are lower than those of any among points 801 to 804.

For example, for point 801, no other set is present in which all of energies Energyⱼ of the respective states Stateⱼ are lower than the set of energies corresponding to point 801. Therefore, the distance from point 801 to the origin O is "0". In this case, the highest rank is assigned to the set of energies Energyⱼ of the respective states Stateⱼ calculated at the k-th iteration and corresponding to point 801.

On the other hand, points 802 to 804 have the same distance from the origin O. For example, for point 802, the only other set in which all energies Energyⱼ of the respective states Stateⱼ are lower than the set corresponding to point 802 is the set corresponding to point 801. Therefore, the distance from the origin O to point 802 is "1". Similarly, for points 803 and 804, the only set in which all energies Energyⱼ of the respective states Stateⱼ are lower than the sets corresponding to points 803 and 804 is the set corresponding to point 801. Therefore, the distance from origin O to points 803 and 804 is "1".

In this case, the rank calculating unit 505, for example, assigns a higher rank to the sets corresponding to points 802 to 804 than to other sets in which the energies Energyⱼ of the lower state Stateⱼ are relatively lower. Among the states State₁ to State_{Ns}, the state with the lowest energy is "State₁". Therefore, the rank calculating unit 505 compares the energies Energy₁ (ground state energy) of the states State₁ of points 802 to 804 with each other.

Here, among points 802 to 804, point 802 has the lowest Energy₁ (ground state energy₎ of the states State₁, point 803 has the second lowest, and point 804 has the third lowest. In this case, the rank calculating unit 505 assigns the second highest rank to the set of energies Energyⱼ of the respective states Stateⱼ in the (k+1)-th iteration, corresponding to point 802.

Furthermore, the rank calculating unit 505 assigns the third-highest rank to the set of energies Energyⱼ of the states Stateⱼ in the (k+2)-th iteration, corresponding to point 803. Furthermore, the rank calculating unit 505 assigns the fourth-highest rank to the set of energies Energyⱼ of the states Stateⱼ in the (k+3)-th iteration, corresponding to point 804.

Regarding points 802 to 804, there may be two or more points with the same energy Energy₁ (ground state energy) of state State₁. In this case, the rank calculating unit 505 compares, the energy Energy₂ (second excited energy) of state State₂ with the second lowest energy, among those two or more points.

Then, based on the comparison result, the rank calculating unit 505 assigns a higher rank to the set of energies Energyⱼ of the respective states Stateⱼ corresponding to the point among the two or more points for which the energy Energy₂ (second excitation energy) of state State₂ is relatively lower than that of the others.

As described, the rank calculating unit 505 can assign different ranks to the sets of energies Energyⱼ corresponding to the states Stateⱼ of the points by comparing and ranking the energies of lower states until differences arise between points having the same distance from the origin O. Furthermore, by starting the comparison from the energy of a lower state, the rank calculating unit 505 can assign a higher rank to sets that represent a more stable state.

### (Specific Example of Energy Analysis Results)

Next, with reference to Fig. 9, a specific example of energy analysis results will be explained. In this example, Ns (the number of states Stateⱼ) is "Ns=3" (energy count 401) and only the best rank (highest rank) is output as energy analysis results.

Fig. 9 is an explanatory diagram depicting a specific example of energy analysis results. In Fig. 9, the energy analysis result 900 has the values of parameters p (p₁, p₂, ...), states State₁ to State₃, and energies Energy₁ to Energy₃ of the states State₁ to State₃. In Fig. 9, State 1 to 3 correspond to the states State₁ to State₃, and Energies 1 to 3 correspond to the energies Energy₁ to Energy₃.

In the example depicted in Fig. 9, only some values of the energy analysis result 900 are specifically depicted. For example, the value of parameter p₁ is "-1.23". The value of parameter p₂ is "2.34". Furthermore, state 2 (State₂) is "(1.0+0.0i, 0.0+1.0i, -1.0+0.0i, 0.0-1.0i, ...)". Also, energy 2 (Energy₂) is "-123.456".

According to the energy analysis result 900, for example, the analyst can determine not only the energy of the ground state (State 1) for the substance-under-analysis but also the energies of the excited states (State 2 and State 3). This enables the analyst to analyze properties such as whether the substance will react or how readily the reaction will proceed.

### (Energy Calculation Process of Excitation Energy Calculation Device 201)

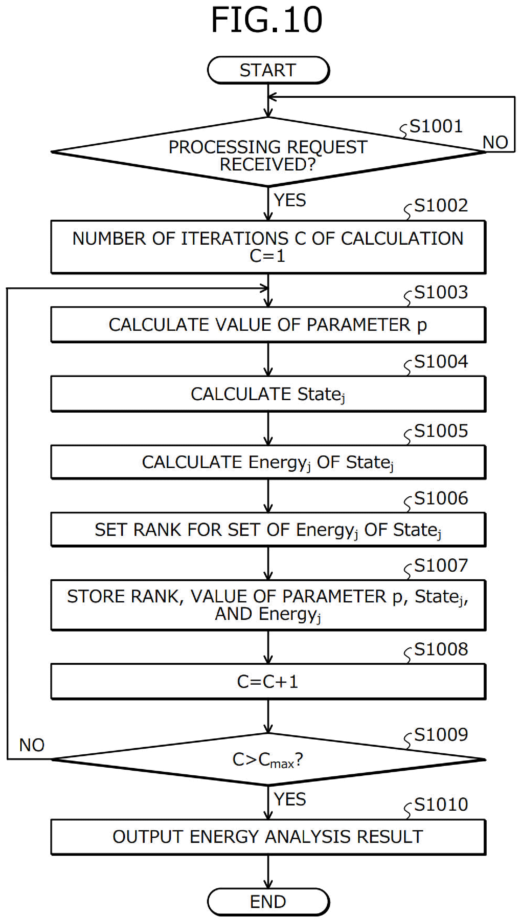

Next, with reference to Fig. 10, a procedure of the energy calculating process of the excitation energy calculating device 201 is described.

Fig. 10 is a flowchart depicting an example of the procedure of the energy calculating process of the excitation energy calculating device 201. In the flowchart depicted in Fig. 10, the excitation energy calculating device 201 first determines whether a processing request from the client device 202 has been received (step S1001). The processing request requests calculation of the energy of an excited state of a substance-under-analysis. The processing request includes, for example, energy calculation setting information 400 as depicted in Fig. 4.

Here, the excitation energy calculating device 201 waits for a processing request to be received (step S1001: NO). When the excitation energy calculating device 201 receives a processing request (step S1001: YES), the excitation energy calculating device 201 initializes the number of iterations C for performing the energy calculation to "C=1" (step S1002).

Next, the excitation energy calculating device 201 calculates the values of parameters p representing respective states Stateⱼ of the substance-under-analysis (step S1003). Specifically, for example, when the number of iterations C is "C=1", the excitation energy calculating device 201 calculates the initial values of parameters p. Furthermore, when the number of iterations C is 2 or greater, the excitation energy calculating device 201 changes the values of parameters p based on the rank assigned at step S1006 and the corresponding values of parameters p for the rank, such that the rank of the set of energies Energyⱼ of the respective states Stateⱼ increases.

Then, the excitation energy calculating device 201, using quantum chemical calculations, calculates each state Stateⱼ based on the values of parameters p (step S1004). Next, the excitation energy calculating device 201 calculates the energies Energyⱼ of the respective states Stateⱼ based on the calculated states Stateⱼ (step S1005).

Then, the excitation energy calculating device 201 assigns a rank to the set of calculated energies Energyⱼ of the respective states Stateⱼ such that in the coordinate system CS, points corresponding to sets relatively closer to the origin O are assigned relatively higher ranks (step S1006).

Specifically, for example, the excitation energy calculating device 201 assigns a rank to the set of energies Energyⱼ of the respective states Stateⱼ calculated this time based on the ranks for other sets stored at step S1007. However, when the number of iterations C is "C=1", the excitation energy calculating device 201 assigns the rank "0" to the set of energies Energyⱼ of the respective states Stateⱼ calculated this time.

Next, the excitation energy calculating device 201 stores associated with one another, the assigned rank, the values of parameters p corresponding to that rank, the calculated state Stateⱼ, and the calculated energies Energyⱼ of respective states Stateⱼ (step S1007).

Next, the excitation energy calculating device 201 increments the number of iterations C (step S1008) and determines whether the number of iterations C is greater than the maximum number of iterations Cₘₐₓ(step S1009). The maximum number of iterations Cₘₐₓ corresponds, for example, to the upper limit 403 of the number of iterations of calculation in the energy calculation setting information 400 included in the processing request.

Here, when the number of iterations C is less than or equal to the upper limit Cₘₐₓ (step S1009: NO), the excitation energy calculating device 201 returns to step S1003. On the other hand, when the number of iterations C is greater than the upper limit Cₘₐₓ (step S1009: YES), the excitation energy calculating device 201 outputs an energy analysis result (step S1010) and terminates the series of processes according to this flowchart.

The energy analysis result includes the energies Energyⱼ of the respective states Stateⱼ having the highest rank assigned at step S1005. The energy analysis result may also include, for example, the values of parameters p for the highest-ranked set of energies Energyⱼ.

This enables the excitation energy calculating device 201 to calculate the energies Energyⱼ of the respective states Stateⱼ (ground state, excited states) of the substance-under-analysis.

As described above, according to the excitation energy calculating device 201 of the embodiment, while repeatedly changing the values of the parameters p for representing each state Stateⱼ of the substance-under-analysis, quantum chemical calculations are performed to calculate each state Stateⱼ based on the values of the parameters p. Based on the calculated states Stateⱼ, the energies Energyⱼ of the respective states Stateⱼ are calculated based on the parameters p. A state Stateⱼ is one of the states State₁ to State_{Ns} which include the ground state and one or more excited states of the substance-under-analysis. At this time, according to the excitation energy calculating device 201, in the coordinate system CS, a rank is assigned to each set of calculated energies Energyⱼ of the respective states Stateⱼ such that the higher ranks are assigned to the sets corresponding to points that are closer to the origin O in the coordinate system CS. Based on the assigned rank and the values of parameters p corresponding to that rank, the values of parameters p can be changed so that the rank of the set of energies Energyⱼ of the respective states Stateⱼ becomes higher. The coordinate system CS is, for example, an orthogonal coordinate system including axes representing the energies Energyⱼ of the respective states Stateⱼ.

Thus, the excitation energy calculating device 201 can calculate the energies of the excited states of the substance-under-analysis. For example, the excitation energy calculating device 201 can accurately calculate the energies of the excited states without using weight coefficients for the energies Energyⱼ of the respective states Stateⱼ, different from the existing SSVQE methods. Therefore, the excitation energy calculating device 201 can reduce the work time and effort necessary for setting weight coefficients, thereby shortening the analysis period for the substance-under-analysis.

Furthermore, according to the excitation energy calculating device 201, by calculating the energies Energyⱼ of the respective states Stateⱼ while repeatedly changing the values of the parameters p, the excitation energy calculating device 201 can output the set of energies Energyⱼ of the respective states Stateⱼ having the highest rank.

Thus, the excitation energy calculating device 201 can output, as energy analysis results (e.g., the energy analysis result 900), solutions that can be considered closest to the actual state of the substance, in a stable state with overall low energy.

Furthermore, according to the excitation energy calculating device 201, for each set of calculated energies Energyⱼ of the respective states Stateⱼ, when another set is present having the same distance from the origin O, the set with the lower state energy can be assigned a higher rank than the other set. Furthermore, according to the excitation energy calculating device 201, when the energy of the lower state is the higher than that of the other set, the energy of the lower state can be assigned a lower rank than the other set.

Thus, the excitation energy calculating device 201 can rank states by comparing and evaluating the energies of lower states in detail when other sets having the same overall energy evaluation (distance from origin O) are present, by determining the lower is the energy of the lower state, the closer the state is to the actual state.

Furthermore, according to the excitation energy calculating device 201, past calculation result data can be obtained. The past calculation result data represents, for each set of energies Energyⱼ of respective states Stateⱼ calculated in the past, a rank assigned such that higher ranks are assigned to the sets corresponding to the points the closer to the origin O in the coordinate system CS, and the values of the parameters p corresponding to the rank. Furthermore, the excitation energy calculating device 201 can assign a rank to the set of the calculated energies Energyⱼ of the respective states Stateⱼ so that higher ranks are assigned to sets corresponding to points that are relatively closer to the origin O in the coordinate system CS, by referring to the obtained past calculation result data. Furthermore, according to the excitation energy calculating device 201, based on the assigned rank, the values of parameters p corresponding to the rank, and the past calculation result data, the values of parameters p can be changed so that the rank of the set of energies Energyⱼ of respective states Stateⱼ becomes higher.

Thus, the excitation energy calculating device 201 can optimize the values of parameters p using past calculation result data. For example, compared to starting from scratch, the excitation energy calculating device 201 can efficiently search for parameter values that yield higher rankings for the calculated set of energies Energyⱼ of respective states Stateⱼ. For instance, even when the time available per day is limited, the excitation energy calculating device 201 can perform energy calculations spread over multiple days, thereby improving user convenience.

Furthermore, based on the assigned rank and the corresponding values of parameters p for the rank, the excitation energy calculating device 201 can use an optimization algorithm to change the values of parameters p. This is achieved by searching for values of parameters p within preset ranges that yield a higher ranking for the set of energies Energyⱼ of respective states Stateⱼ to be calculated next.

Thus, the excitation energy calculating device 201 can efficiently search for the values of parameters p using existing optimization algorithms such as the Nelder-Mead method or Powell's conjugate gradient method.

Therefore, the excitation energy calculating device 201 enables the calculation of not only the ground state energy but also the energies of excited states of a substance-under-analysis thereby enabling the analysis of properties such as whether a reaction occurs or how readily a reaction proceeds. For example, an analyst can determine the energy difference between different states based on the energies Energyⱼ of the respective states Stateⱼ and may thereby know that light corresponding to this energy difference is absorbed or emitted, which can be utilized for applications such as coloring fireworks.

The energy calculating method described in the present embodiment may be implemented by executing a prepared program on a computer such as a personal computer and a workstation. The program is stored on a non- transitory, computer-readable recording medium such as a hard disk, a flexible disk, a compact disc read-only memory (CD-ROM), a magneto-optical (MO) disc, and a digital versatile disc (DVD), read out from the computer-readable medium, and executed by the computer. The program may be distributed

The information processing device 100 (excitation energy calculating device 201) described in the present embodiment can be realized by an application specific integrated circuit (ASIC) such as a standard cell or a structured ASIC, or a programmable logic device (PLD) such as a field- programmable gate array (FPGA).

### EXPLANATIONS OF LETTERS OR NUMERALS

100 Information Processing Device
200 Information Processing System
201 Excitation Energy Calculating Device
202 Client Device
210 Network
300 Bus
301 CPU
302 Memory
303 Network Interface
304 Recording Medium Interface
305 Recording Medium
400 Energy Calculation Settings
401 Energy Count
402 Parameter Ranges
403 Upper Limit of Iterations
500 Controller
501 Obtaining Unit
502 Parameter Calculating Unit
503 State Calculating Unit
504 Energy Calculating Unit
505 Ranking Calculating Unit
506 Output Unit
510 Storage Unit
600 Calculation Result Table
601, 602, 603 Calculation Results
801, 802, 803, 804 Points
900 Energy Analysis Results
CS Coordinate System
O Origin
p, p₁, p_{2,}..., p_{Np} Parameters
State₁ to State_{Ns,} Stateⱼ States
Energyⱼ Energy

## Claims

1. An energy calculating program for causing a computer to execute a process, the process comprising:
while changing a value of a parameter for expressing each of a plurality of states including a ground state and one or more excited states of a substance,
calculating by quantum chemical calculation, the plurality of states, based on the value of the parameter; and
calculating based on the calculated plurality of states, a plurality of energy sets of the plurality of states, wherein
the changing the value of the parameter includes:
assigning a rank to each of the calculated plurality of energy sets so that in a coordinate system including axes representing the plurality of energy sets, the rank of a set that is among the plurality of energy sets and corresponds to a point relatively closer to an origin of the coordinate system is relatively higher, and
changing the value of the parameter based on the assigned rank and the value of the parameter corresponding to the rank so that the rank of the set becomes higher.

2. The energy calculating program according to claim 1, the process further comprising outputting among the plurality of energy sets, the set to which a highest rank is assigned as a result of calculating the plurality of energy sets while changing the value of the parameter.

3. The energy calculating program according to claim 1, wherein, when a first set and a second set of the plurality of energy sets have a same distance from the origin, the assigning includes assigning a higher rank to the first set when an energy of a lower state thereof is lower than that of the second set, and assigning a lower rank to the first set when the energy of the lower state thereof is higher than that of the second set.

4. The energy calculating program according to claim 1, the process further comprising obtaining past calculation result data representing, for each of a plurality of past energy sets of the plurality of states, calculated in the past, the rank assigned to the each of the plurality of past energy sets and the value of the parameter corresponding to the rank, in the past calculation result data, the rank being assigned to the each of the plurality of past energy sets so that a set that is among the plurality of past energy sets and corresponds to a point relatively closer to the origin of the coordinate system is assigned a relatively higher rank, wherein
the assigning includes referring to the obtained past calculation result data and assigning the rank to the each of the calculated plurality of energy sets, and
the changing includes changing based on the assigned rank, the value of the parameter corresponding to the rank, and the past calculation result data, the value of the parameter so that the rank to be assigned to the each of the calculated plurality of energy sets increases.

5. The energy calculating program according to claim 1, wherein,
the changing includes using an optimization algorithm and changing based on the assigned rank and the value of the parameter corresponding to the rank, the value of the parameter by searching within a preset range so that the rank of the energy set when calculated next time increases.

6. An energy calculating method executed by a computer, the method comprising:
while changing a value of a parameter for expressing each of a plurality of states including a ground state and one or more excited states of a substance,
calculating by quantum chemical calculation, the plurality of states, based on the value of the parameter; and
calculating based on the calculated plurality of states, a plurality of energy sets of the plurality of states, wherein
the changing the value of the parameter includes:
assigning a rank to each of the calculated plurality of energy sets so that in a coordinate system including axes representing the plurality of energy sets, the rank of a set that is among the plurality of energy sets and corresponds to a point relatively closer to an origin of the coordinate system is relatively higher, and
changing the value of the parameter based on the assigned rank and the value of the parameter corresponding to the rank so that the rank of the set becomes higher.

7. An information processing device comprising a controller, the controller being configured to:
while changing a value of a parameter for expressing each of a plurality of states including a ground state and one or more excited states of a substance,
calculate by quantum chemical calculation, the plurality of states, based on the value of the parameter; and
calculate based on the calculated plurality of states, a plurality of energy sets of the plurality of states, wherein
the changing the value of the parameter includes:
assigning a rank to each of the calculated plurality of energy sets so that in a coordinate system including axes representing the plurality of energy sets, the rank of a set that is among the plurality of energy sets and corresponds to a point relatively closer to an origin of the coordinate system is relatively higher, and
changing the value of the parameter based on the assigned rank and the value of the parameter corresponding to the rank so that the rank of the set becomes higher.
